(19) Europäisches Patentamt
European Patent Office
Office européen des brevets

(11) EP 1 634 606 A1

(12) # EUROPEAN PATENT APPLICATION
published in accordance with Art. 158(3) EPC

(43) Date of publication:
**15.03.2006 Bulletin 2006/11**

(21) Application number: **04724756.4**

(22) Date of filing: **31.03.2004**

(51) Int Cl.:
***A61K 45/00*** *(1985.01)* ***A61K 31/443*** *(2000.01)*
***A61P 11/00*** *(2000.01)* *C07D 405/06* *(1990.01)*

(86) International application number:
**PCT/JP2004/004601**

(87) International publication number:
**WO 2004/096274 (11.11.2004 Gazette 2004/46)**

(84) Designated Contracting States:
**AT BE BG CH CY CZ DE DK EE ES FI FR GB GR HU IE IT LI LU MC NL PL PT RO SE SI SK TR**

(30) Priority: **31.03.2003 JP 2003094505**

(71) Applicant: **KYOWA HAKKO KOGYO CO., LTD.**
**Chiyoda-ku,**
**Tokyo 100-8185 (JP)**

(72) Inventors:
- **OHSHIMA, Etsuo**
  **c/o Pharmaceutical Research Center**
  **Nagaizumi-cho Sunto-Gun Shizuoka411-8731 (JP)**
- **YAMAGUCHI, Kazuo**
  **Kanagawa 228-0827 (JP)**
- **MANABE, Haruiko**
  **c/o Pharmaceutical Research Center**
  **Nagaizumi-cho Sunto-Gun Shizuoka411-8731 (JP)**
- **MIE, Motoya**
  **c/o Pharmaceutical Research Center**
  **Nagaizumi-cho Sunto-Gun Shizuoka411-8731 (JP)**
- **TAHARA, H.**
  **c/o Pharmaceutical Research Center**
  **Nagaizumi-cho Sunto-Gun Shizuoka411-8731 (JP)**
- **ISHIKAWA, Y.**
  **c/o Pharmaceutical Research Center**
  **Nagaizumi-cho Sunto-Gun Shizuoka411-8731 (JP)**

(74) Representative: **Casalonga, Axel**
**BUREAU D.A. CASALONGA - JOSSE**
**Paul-Heyse-Strasse 33**
**80336 München (DE)**

(54) **DRUG FOR AIRWAY ADMINISTRATION**

(57) An agent for intra-airway administration comprising a compound having a PDE-IV inhibitory activity or the pharmaceutically acceptable salt thereof as an active ingredient which shows its concentration in lung tissues 350-times or more higher than its concentration in plasma when administered into the airway is provided. An agent for intra-airway administration, wherein the compound having a PDE-IV inhibitory activity is, for example, a benzofuran derivative or a 1,3-benzodioxole derivative is provided.

Fig. 1

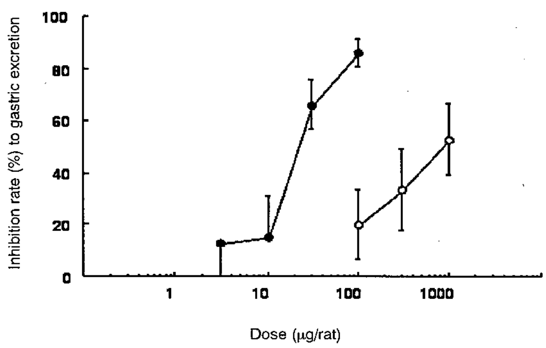


EP 1 634 606 A1

## Description

Technical Field

The present invention relates to an agent for intra-airway administration comprising a compound having a phosphodiesterase (PDE)-IV inhibitory activity or a pharmaceutically acceptable salt thereof as an active ingredient.

Background Art

A PDE-IV inhibitor containing a compound having a phosphodiesterase-IV inhibitory activity or a pharmaceutically acceptable salt thereof as an active ingredient (hereinafter, referred to as a PDE-IV inhibitor) has been known to be useful for respiratory diseases and, for example, the following reports have been made.

(i) In BN rats, a PDE-IV inhibitor inhibits infiltration of eosinophils into the lung induced by antigen [*J. Pharmacol. Exp. Ther.,* volume 297, pages 280-290 (2001); *Bioorg.Med. Chem. Lett.,* volume 8, pages 3229-3234 (1998); *Br J. Pharmacol.,* volume 113, pages 1423-1431 (1994)].
(ii) In BN rats, a PDE-IV inhibitor suppresses airway constriction induced by antigen [*J. Pharmacol. Exp. Ther.,* volume 297, pages 280-290 (2001); *Br. J. Pharmacol.,* volume 113, pages 1423-1431 (1994)].

With regard to side effects of the PDE-IV inhibitor, influence on digestive organs such as vomiting has been known, and the following reports have been made.

(i) A PDE-IV inhibitor promotes the secretion of gastric acid in rats [*J. Pharmacol. Expt Ther.,* volume 292, pages 647-653 (2000)].
(ii) A PDE-IV inhibitor suppresses the gastric excretin in rats *[Bioorg.Med. Chem. Lett.,* volume 12, pages 653-658 (2002)].
(iii) A PDE-IV inhibitor induces vomiting in dogs by oral or intravenous administration [*Eur. J. Pharmacol.*, volume 286, pages 281-290 (1995) *; J. Med. Chem.,* volume 42, pages 1088-1099 (1999); *J. Med. Chem.* volume 42, pages 4216-4223 (1998) ; *Lab. Anim. Sci.,* volume 45, pages 647-651 (1995)].
(iv) A PDE-IV inhibitor induces vomiting in sunks by the oral or intraperitoneal administration [*J. Med. Chem.,* volume 40, pages 3248-3253 (1997)].

From the above, it has been recognized that it is necessary to separate, in a PDE-IV inhibitor, the pharmacological effect to the airway system from that to the digestive system.
In the meanwhile, benzofuran derivatives, 1,3-benzodioxole derivatives and the like having a PDE-IV inhibitory activity, such as 7-[2-(3,5-dichloro-4-pyridyl)-1-oxoethyl]-4-methoxy-spiro[1,3-benzodioxole-2,1'-cyclopentane] or a pharmaceutically acceptable salt thereof, and the like have been known (WO 96/36624).

Disclosure of the Invention

An object of the present invention is to provide an agent for intra-airway administration comprising a compound having a PDE-IV inhibitory activity or a pharmaceutically acceptable salt thereof as an active ingredient, and in addition, being able to separate the pharmacological effect to airway system from that to the digestive system.
The present invention relates to the following (1) to (22).

(1) An agent for intra-airway administration comprising a compound having a PDE-IV inhibitory activity or a pharmaceutically acceptable salt thereof as an active ingredient which shows its concentration in lung tissues 350-times or more higher than its concentration in plasma when administrated into the airway.
(2) An agent for intra-airway administration comprising a compound having a PDE-IV inhibitory activity or a pharmaceutically acceptable salt thereof as an active ingredient which shows its concentration in lung tissues 500-times or more higher than its concentration in plasma when administrated into the airway.
(3) An agent for intra-airway administration comprising a compound having a PDE-IV inhibitory activity or a pharmaceutically acceptable salt thereof as an active ingredient which shows its concentration in lung tissues 1,000-times or more higher than its concentration in plasma when administrated into the airway.
(4) An agent for intra-airway administration comprising a compound having a PDE-IV inhibitory activity or a pharmaceutically acceptable salt thereof as an active ingredient which shows its concentration in lung tissues 2,000-times or more higher than its concentration in plasma when administered into the airway.
(5) The agent for intra-airway administration according to any one of (1) to (4), wherein the compound having a

PDE-IV inhibitory activity is a compound represented by formula (I)

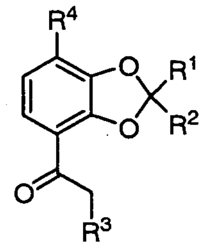

(I)

(wherein, $R^1$ and $R^2$ are the same or different and each represents lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

(6) The agent for intra-airway administration according to (5), wherein $R^3$ is substituted or unsubstituted pyridyl.

(7) The agent for intra-airway administration according to (1), wherein the compound having a PDE-IV inhibitory activity is 7-[2-(3,5-dichloro-4-pyridyl)-1-oxoethyl]-4-methoxy-spiro[1,3-benzodioxole-2,1'-cyclopentane] represented by formula (II):

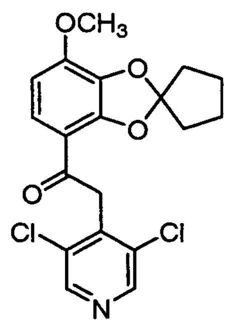

(II)

(8) A method of treating and/or preventing a respiratory disease, which comprises administrating an effective amount of a compound having a PDE-IV inhibitory activity or a pharmaceutically acceptable salt thereof into the airway, which shows its concentration in lung tissues 350-times or more higher than its concentration in plasma when administered into the airway.

(9) A method of treating and/or preventing a respiratory disease, which comprises administrating an effective amount of a compound having a PDE-IV inhibitory activity or a pharmaceutically acceptable salt thereof into the airway, which shows its concentration in lung tissues 500-times or more higher than its concentration in plasma when administered into the airway.

(10) A method of treating and/or preventing a respiratory disease, which comprises administrating an effective amount of a compound having a PDE-IV inhibitory activity or a pharmaceutically acceptable salt thereof into the airway, which shows its concentration in lung tissues 1,000-times or more higher than its concentration in plasma when administered into the airway.

(11) A method of treating and/or preventing a respiratory disease, which comprises administrating an effective amount of a compound having a PDE-IV inhibitory activity or a pharmaceutically acceptable salt thereof into the airway, which shows its concentration in lung tissues 2,000-times or more higher than its concentration in plasma when administered into the airway.

(12) The method of treating and/or preventing a respiratory disease according to any one of (8) to (11), wherein the compound having a PDE-IV inhibitory activity is a compound represented by formula (I)

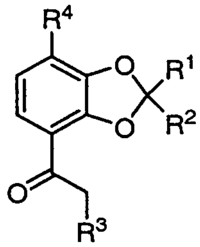

(I)

(wherein, $R^1$ and $R^2$ are the same or different and each represents lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

(13) The method of treating and/or preventing a respiratory disease according to (12), wherein $R^3$ is substituted or unsubstituted pyridyl.

(14) The method of treating and/or preventing a respiratory disease according to (8), wherein the compound having a PDE-IV inhibitory activity is 7-[2-(3,5-dichloro-4-pyridyl)-l-oxoethyl]-4-methoxy-spiro-[1,3-benzodioxole-2,1'-cyclopentane] represented by formula (II):

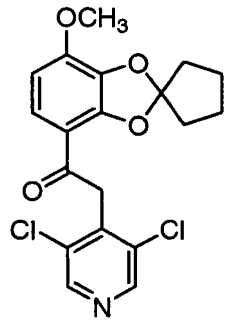

(II)

(15) The method of treating and/or preventing a respiratory disease according to any one of (8) to (14), wherein the respiratory disease is a disease selected from the group consisting of bronchial asthma, chronic obstructive pulmonary disease (COPD), pulmonary emphysema, chronic bronchitis, pulmonary fibrosis, pulmonary hypertension and eosinophilic pneumonia.

(16) Use of a compound having a PDE-IV inhibitory activity or a pharmaceutically acceptable salt thereof which shows its concentration in lung tissues 350-times or more higher than its concentration in plasma when administered into the airway, for the manufacture of an agent for intra-airway administration.

(17) Use of a compound having a PDE-IV inhibitory activity or a pharmaceutically acceptable salt thereof which shows its concentration in lung tissues 500-times or more higher than its concentration in plasma when administered into the airway, for the manufacture of an agent for intra-airway administration.

(18) Use of a compound having a PDE-IV inhibitory activity or a pharmaceutically acceptable salt thereof which shows its concentration in lung tissues 1,000-times or more higher than its concentration in plasma when administered into the airway, for the manufacture of an agent for intra-airway administration.

(19) Use of a compound having a PDE-IV inhibitory activity or a pharmaceutically acceptable salt thereof which shows its concentration in lung tissues 2,000-times or more higher than its concentration in plasma when administered into the airway, for the manufacture of an agent for intra-airway administration.

(20) Use according to any one of (16) to (19), wherein the compound having a PDE-IV inhibitory activity is a compound represented by formula (I)

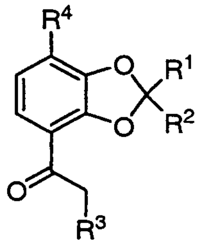

(I)

(wherein, $R^1$ and $R^2$ are the same or different and each represents lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

(21) Use according to (20), wherein $R^3$ is substituted or unsubstituted pyridyl.

(22) Use according to (16), wherein the compound having a PDE-IV inhibitory activity is 7-[2-(3,5-dichloro-4-pyridyl)-1-oxoethyl]-4-methoxy-spiro-[1,3-benzodioxole-2,1'-cyclopentane] represented by formula (II):

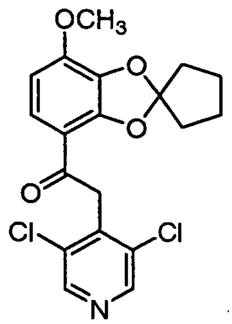

(II)

In this specification, the term airway system means all organs participated in respiration, and, to be more specific, they are nasal cavity, paranasal sinus, oral cavity, throat, tonsil, trachea, bronchus, alveoli, and the like. Blood vascular systems attached thereto are also included. Furthermore, the term airway means a path for breathing, and, to be more specific, they are inside of nasal, sinus, mouth, throat, bronchus, bronchial branch, pulmonary alveoli, and the like.

According to the agent for intra-airway administration of the present invention, for example, treatment of respiratory diseases can be made. Examples of the respiratory disease include respiratory disease accompanied by constriction of bronchial smooth muscle, respiratory disease accompanied by constriction of airway vascular system, respiratory diseases accompanied by inflammation, respiratory diseases accompanied by secretion of mucus, respiratory disease accompanied by reversible or irreversible organic changes of airway paries, respiratory diseases accompanied by reversible or irreversible organic changes of airway vascular system, respiratory diseases accompanied by reversible or irreversible organic changes of pulmonary alveoli, respiratory diseases accompanied by reversible or irreversible organic changes of nasal cavity and respiratory diseases accompanied by reversible or irreversible organic changes of paranasal sinus. More specific examples include bronchial asthma, COPD, pulmonary emphysema, chronic bronchitis, pulmonary fibrosis, pulmonary hypertension, eosinophilic pneumonia, allergic rhinitis, eosinophilic sinusitis, chronic or acute sinusitis, pharyngitis, tonsillitis and the like.

Examples of the method of administrating into the airway of the agent for intra-airway administration of the present invention include intra-airway infusion, intra-airway inhalation and the like. Examples of more specific dosage form include nasal drops, dry powder preparation, sprays preparation of solution, sprays preparation of suspension and the like.

With regard to a substance which is used as an active ingredient for the agent for intra-airway administration of the present invention, any substance may be used so far as it is a compound having a PDE-IV inhibitory activity or a pharmaceutically acceptable salt thereof, where the AUC in lung tissues (concentration in lung tissues) is 350-fold or more higher than the AUC in plasma (concentration in plasma) when dose of 4 mg/kg is administered, by the same method which will be mentioned in Test Example 1 later, into airway of test animals (SD strain rats) and each AUC in

lung tissues and plasma (area under curve of concentration in lung tissues and plasma versus time, respectively) is measured. The compounds having a PDE-IV inhibitory activity or a pharmaceutically acceptable salts thereof, where the AUC in lung tissues is preferably 500-fold or more, more preferably 1, 000-fold or more and, still more preferably 2,000-fold or more higher than the AUC in plasma, are included.

More specific examples of the substance which is used as an active ingredient of the agent for intra-airway administration of the present invention include the compounds having the following properties (a) to (f), although the substance used as an active ingredient for the agent for intra-airway administration of the present invention are not limited thereto. Hereinafter, the compound having a PDE-IV inhibitory activity or a pharmaceutically acceptable salt thereof shall be called collectively "substance".

(a) The property that absorption of a substance via lung being administered into airway is very slow, and in addition, after transferred to circulation blood, it is quickly subjected to hepatic metabolism to be inactivated while a substance swallowed into a digestive tract is absorbed from intestinal tract and then quickly metabolized and inactivated in the liver in the same manner;
(b) The property that a substance which is administered into airway shows a high affinity to protein which is inherent to the lung and its concentration equilibrium greatly leans to the lung;
(c) The property that crystals of a substance administered into airway are slowly dissolved and absorbed via the lung while the substance swallowed into a digestive tract is not absorbed from the intestinal tract but is just excreted into feces;
(d) The property that absorption via the lung of a substance which is administered into airway is very slow, and in addition, after it is transferred to circulation blood, it quickly forms a conjugate to be excreted;
(e) The property that absorption via the lung of a substance which is administered into airway is very slow, and in addition, after it is transferred to circulation blood, it is quickly metabolized and inactivated by an enzyme in blood; and
(f) The property that a substance is a prodrug (a precursor for activity), and the substance which is administered into airway is metabolized and activated by an enzyme which is specific in the lung while a substance swallowed into a digestive tract is not metabolized and is not absorbed from intestinal tract as well but is directly excreted into feces.

Specific examples of the compound having a PDE-IV inhibitory activity used as an active ingredient of the agent for intra-airway administration of the present invention include 1,3-benzodioxole derivatives, benzofuran derivatives and the like mentioned, for example, in WO 96/36624, WO 99/16768 and the like. More specific examples include a compound represented by formula (I)

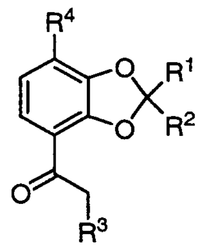

(I)

(wherein, $R^1$, $R^2$, $R^3$ and $R^4$ each has the same meaning as defined already), preferably the compound that $R^3$ is a substituted or unsubstituted pyridyl, and, further preferably, 7-[2-(3,5-dichloro-4-pyridyl)-1-oxoethyl]-4-methoxy-spiro-[1,3-benzodioxole-2,1'-cyclopentane] represented by formula (II):

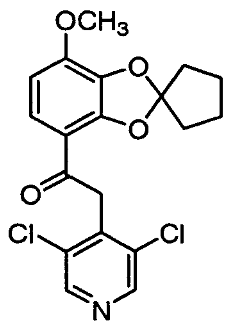

( II )

However, the compounds having a PDE-IV inhibitory activity used as an active ingredient for the agent for intra-airway administration of the present invention are not limited thereto.

Hereinafter, each of the compounds represented by the formula (I) and the formula (II) will be referred to as Compound (I) and Compound (II), respectively. That will be also the same for the compounds bearing other formula numbers.

In the definition for each of the groups in the formula (I), with regard to the lower alkyl and the lower alkyl moiety in the lower alkoxy, a linear or branched alkyl having 1 to 8 carbon atom(s) may be listed, and, for example, methyl, ethyl, propyl, isopropyl, butyl, isobutyl, sec-butyl, tert-butyl, pentyl, neopentyl, hexyl, heptyl, octyl and the like may be listed.

With regard to the saturated carbon ring formed together with the adjacent carbon atom, a cycloalkane having 3 to 8 carbon atoms may be listed, and, for example, cyclopropane, cyclobutane, cyclopentane, cyclohexane, cycloheptane, cyclooctane and the like may be listed.

With regard to the aromatic heterocyclic group, a five-or six-membered monocyclic aromatic heterocyclic group containing at least one atom selected from a nitrogen atom, an oxygen atom and a sulfur atom may be listed, and, for example, pyridyl, pyrazinyl, pyrimidinyl, pyridazinyl, pyrrolyl, pyrazolyl, triazolyl, tetrazolyl, imidazolyl, oxazolyl, thiazolyl, thienyl, furyl and the like may be listed.

With regard to the substituents in the substituted aromatic heterocyclic group and the substituted pyridyl, for example, one to three substituent(s) such as lower alkoxy, halogen and the like, which are the same or different, may be listed. The lower alkoxy mentioned here is the same as that mentioned already and the halogen is each of fluorine, chlorine, bromine and iodine atoms.

The pharmaceutically acceptable salts of the compound having a PDE-IV inhibitory activity includes pharmaceutically acceptable acid addition salts, metal salts, ammonium salts, organic amine addition salts, amino acid addition salts and the like.

With regard to the pharmaceutically acceptable acid addition salt of the compound having a PDE-IV inhibitory activity, inorganic acid salts such as a hydrochloride, a sulfate, a nitrate, a phosphate and the like, and organic acid salts such as an acetate, a maleate, a fumarate, a citrate and the like may be listed. With regard to the pharmaceutically acceptable metal salts, alkali metal salts such as a sodium salt, a potassium salt and the like, alkaline earth metal salts such as a magnesium salt, a calcium salt and the like, an aluminum salt, a zinc salt, and the like may be listed. With regard to the pharmaceutically acceptable ammonium salts, salts such as an ammonium salt, a tetramethylammonium salt and the like may be listed. With regard to the pharmaceutically acceptable organic amine addition salts, addition salts of morpholine, piperidine or the like may be listed. With regard to the pharmaceutically acceptable amino acid addition salts, addition salts of glycine, phenylalanine, lysine, aspartic acid, glutamic acid or the like may be listed.

Now, the process for the preparation of Compound (I) will be described.

Compound (I) is able to be prepared by the process mentioned in WO 96/36624.

Among Compound (I), stereoisomers such as tautomer may be present, however, all possible isomers and the mixture thereof including the above may be used for the agent for intra-airway administration of the present invention.

In order to prepare the salt of Compound (I), the salt of Compound (I) itself may be just purified when Compound (I) is prepared in a form of the salt, while when it is prepared in a free form, Compound (I) may be dissolved or suspended in an appropriate solvent and then the salt of Compound (I) may be isolated and purified by addition of acid or base.

Compound (I) and the pharmaceutically acceptable salt may also be present in a form of an adduct with water or with various solvents and such an adduct may be also used for the agent for intra-airway administration of the present invention.

Now, the effect of the agent for intra-airway administration of the present invention will be specifically described by way of Test Examples.

Test Example 1: Changes in concentration in lung tissues and in plasma upon intra-airway administration (in SD rats)
Male rats of SD strain (Nippon Charles Liver, Kanagawa) were bred under the condition of temperature of 23 ± 1°C and humidity of 55 ± 5%, and those where body weight was 210 to 240 g at seven weeks age were used for the test. Administration of the test compound (Compound (II)) was conducted under a non-fasting condition, and during the test, solid feed (F-2; Funahashi Nojo, Chiba) and tap water were freely taken by them. Case numbers for the test was made n = 2 for each stage.

<Preparation of the administering solution>

According to a conventional method, the preparations of Compound (II) and RP 73401 being known as a PDE-IV inhibitor

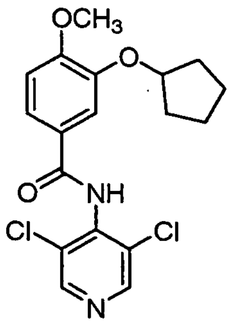

RP73401

[*J. Med. Chem.,* volume 37, pages 1696-1703 (1994)] were prepared (lactose/Compound (II) or RP 73401 = 5/1), and each of them was suspended in 0.125 weight/volume % tyloxapol (Alevaire: registered trade mark, Nippon Shoji, Osaka). The resulting suspension was further diluted with 0.125 weight/volume % tyloxapol containing 0.25 weight/volume % of lactose (Pharmatose 325 M; registered trade mark, DMV International, Veghel, The Nethelands) (a solvent for administration), and a suspension for administration of Compound (II) or RP 73401 having an desired concentration was prepared.

<Intrabronchial Administration>

Each of Compound (II) and RP 73401 was intrabronchially administered at a dose of 4 mg/kg, respectively. The solvent for administration or the suspension for administration of Compound (II) or RP 73401 was intrabronchially administered to each of right and left bronchi of an ether-anesthetized rat at a dose of 100 μL (200 μL in total) using a disposable oral sound (for mice; Fuchigami Kikai, Kyoto).

<Collection of blood and extirpation of organs>

At each of stages of 0.25, 0.5, 1, 2 and 4 hours after the intrabronchial administration, abdomen of rat under a light anesthetization with ether was incised, and about 1 mL of blood was collected from a descending vena cava using a heparin-treated syringe (24G; 1mL; Thermo, Tokyo). Plasma was separated from the blood using a cooling centrifugal separator and stored by freezing at -20°C until the measurement. After washing the pulmonary alveoli, the bronchus was resected to collect the lung. The wet weight of the lung was measured and the lung was stored by freezing.

<Preparation of plasma sample and measurement of AUC in plasma (concentration in plasma)>

To 100 μL of the plasma collected hereinabove was added a 200 μL of methanol solution of 25 μg/mL 7-[2-(3,5-dichloro-4-pyridyl)-1-oxoethyl]-4-methoxy-spiro-[1,3-benzodioxole-2,1'-cyclohexane] (an internal standard substance), and the mixture was stirred. The mixture was allowed to stand for 10 minutes on ice, followed by centrifuged, and then 200 μL of the supernatant was dried under a nitrogen flow. The resulting solid was dissolved in 200 μL of methanol and centrifuged. The resulting supernatant was subjected to centrifugal filtration using a centrifugal filtration unit ultrafree (C3GV; 0.22 μm; Nippon Millipore, Tokyo). The filtrate (100 μL) was used as a plasma sample and concentrations of Compound (II) and RP 73401 were measured using a mass spectrometer (LC/MS/MS).

<Preparation of lung samples and measurement of AUC in lung tissues (concentration in lung tissues)>

In an immunotube (15 mL: Nunc, Glostrup, Denmark), to the lung prepared hereinabove was added the internal standard substance in which the concentration to the lung tissues was previously made 10 μg/g lung tissues, and the mixture was dried under a nitrogen flow. To the resulting solid was added pure water (Milli-Q SP Reagent Water System: Nippon Millipore, Tokyo) in an amount of one-fold to the wet weight followed by subjecting to homogenizing using a homogenator (Ikemoto Rika Kogyo, Tokyo). A two-fold amount of methanol was further added thereto and the mixture was homogenized, allowed to stand for 10 minutes on ice and centrifuged. The supernatant (300 μL) was taken into a microtube and centrifuged once again. The resulting supernatant (100 μL) was used as a lung sample, and concentrations of Compound (II) and RP 73401 were measured by using of a high-performance liquid chromatography (HPLC).

When Compound (II) was intrabronchially administered, the resulting AUC in lung tissues (concentration in lung tissues) was 3,640 mgh/mL and AUC in plasma (concentration in plasma) was 0.733 mgh/mL, and the concentration in lung tissues showed the value as high as 4,970-fold as compared with the concentration in plasma. Incidentally, the biological half-life ($T_{1/2}$) was 3.81 hours.

On the other hand, when RP 73401 was intrabronchially administered, the resulting AUC in lung tissues (concentration in lung tissues) was 248 mgh/mL and AUC in plasma (concentration in plasma) was 0.826 mgh/mL, and the concentration in lung tissues showed the value as high as 300-fold as compared with the concentration in plasma. Incidentally, the biological half-life ($T_{1/2}$) was 0.341 hour.

Test Example 2: Effect to antigen-induced eosinophil infiltration or antigen-induced neutrophil infiltration by intrabronchial administration (BN rats)

The test was conducted in accordance with a known method [***Pulmonary Pharmacology***, volume 8, pages 83-89 (1995)].

Male BN rats of six weeks age (Nippon Charles Liver) were used for the test. Five to six rats were put in each plastic cage in a breeding room where the room temperature was 19 to 25°C, humidity was 30 to 70% and a daily illumination was 12 hours (from 7 a.m. to 7 p.m.), and breeding was conducted where commercially available solid feed and water were freely taken by the rats.

A suspension (1 mL) of 1 mg of ovalbumin (OVA; manufactured by Sigma) and 200 mg of aluminum hydroxide (manufactured by Wako Pure Chemical) in 1 mL of a physiological saline (manufactured by Otsuka Pharmaceutical) was subcutaneously administered to each rat, and then 0.5 mL of a suspension of killed ***Virdetella pertussis*** ($2 \times 10^{10}$ cells/mL physiological saline solution; manufactured by Kaken Pharmaceutical) was intraperitoneally administered to conduct active sensitization. After 14 days from the active sensitization, the rats were placed in a plastic chamber (30 x 50 x 30 cm) and subjected to an antigenic exposure by nebulizing of an 1 weight/volume % solution of OVA in a physiological saline for 10 minutes. For the nebulizing, an ultrasonic nebulizer (manufactured by Omron) was used.

By the same manner as in Test Example 1, a suspension for administration of Compound (II) was prepared and intrabronchially administered to the sensitized rats.

Before 30 minutes of the antigenic exposure, the solvent for administration or the suspension for administration of Compound (II) was intrabronchially administered to the sensitized rats at a dose of 100 μL to each of right and left bronchi (200 μL in total). A group where a suspension for administration of Compound (II) was intrabronchially administered to the sensitized rat and an 1 weight/volume % OVA-physiological saline solution was nebulized, was called a Compound (II)-administered group; a group where the solvent for administration was intrabronchially administered to the sensitized rat and an 1 weight/volume % OVA-physiological saline solution was nebulized, was called a solvent-administered group; and a group where the solvent for administration was intrabronchially administered and a physiological saline was nebulized, was called a physiological saline group.

After 24 hours of the antigenic exposure or of the nebulizing with a physiological saline, a broncho-alveolar lavage (BAL) was conducted. Thus, a rat was anesthetized by an intraperitoneal administration of pentobarbital (manufactured by Dainippon Pharmaceutical) and pulmonary alveoli cavity was washed with a physiological saline via an airway cannula to prepare a broncho-alveolar lavage fluid (BALF).

The BALF was centrifuged under the condition of 950 rpm (200 x g) at 4°C for 10 minutes, the precipitated cells were suspended in 1 mL of a physiological saline, and total leukocyte numbers were measured by an automatic blood corpuscle counter (manufactured by Nippon Koden). Smeared samples of cells were also prepared using Cytospin III (manufactured by Shandon). After the smeared sample was subjected to a Giemsa staining, 500 cells were observed under an optical microscope, numbers of eosinophils and neutrophils were counted and their ratios were calculated. Number of eosinophils and number of neutrophils were calculated by multiplying the total leukocyte numbers by each ratio of number of eosinophils and number of neutrophils, respectively. Inhibition rate (%) to increases of number of eosinophils and number of neutrophils respectively in BALF was calculated according to the following.

$$\text{Inhibition ratio (\%)} = \frac{A - B}{A - C} \times 100$$

A: Numbrer of eosinophils or neutrophils in the solvent-administering group
B: Numbrer of eosinophils or neutrophils in the Compound (II)-administered group
C: Numbrer of eosinophils or neutrophils in the physiological saline group

The results are shown in Table 1 and Table 2, respectively. Incidentally, each of the values for number of eosinophils and number of neutrophils is shown in "mean value ± standard error".

Table 1

| Administered Group | Dose (μg/rat) | Number of eosinophisl ($10^6$/BALF) | Inhibition rate (%) |
|---|---|---|---|
| Physiological Saline | - | 0.02 ± 0.00 | - |
| Solvent | - | 1.16 ± 0.02* | - |
| Compound (II) | 30 | 0.09 ± 0.03 | 50 |
| Compound (II) | 100 | 0.04±0.01## | 86 |
| *: p < 0.001 (as compared with a physiological saline group; Aspin-Welch test)<br>##: p < 0.001 (as compared with a solvent-administered group; Dunnett test) | | | |

Table 2

| Administered Group | Dose (μg/rat) | Number of neutrophils ($10^6$/BALF) | Inhibition rate (%) |
|---|---|---|---|
| Physiological Saline | - | 0.26 ± 0.05 | - |
| Solvent | - | 1.11 ± 0.13 | - |
| Compound (II) | 30 | 0.64 ± 0.14 | 56 |
| Compound (II) | 100 | 0.43 ± 0.06# | 80 |
| #: p < 0.01 (as compared with a solvent-administered group; Dunnett test) | | | |

The number of eosinophils and number of neutrophils in BALF are decreased by intrabronchial administration of Compound (II) to the BN rats, and infiltration of eosinophils and infiltration of neutrophils into bronchial pulmonary alveoli induced by antigen were also inhibited. Since this evaluation system has been known, for example, to represent a part of pathosis of human asthma, Compound (II) is considered to be effective for human asthma.
On the other hand, inhibitory effect of RP 73401 to infiltration of eosinophils and neutrophils to bronchial pulmonary alveoli induced by antigen was investigated by the same manner as in the above test. As the results, RP 73401 showed a significant inhibitory activity to infiltration of eosinophils and infiltration of neutrophils induced by antigen at the dose of 30 to 100 μg/rat each.
Test Example 3: Inhibitory activity for gastric excretion by intrabronchial administration (SD rats)
Male SD rats (six weeks age; body weight upon purchase was 180 to 200 g; Nippon SLC) were used for the test. The rats were bred in an animal room where room temperature was 23 ± 1°C, humidity was 55 ± 5% and solid feed and drinking water were made freely taken by them.
Suspensions for administration of Compound (II) and RP 73401 were prepared by the same manner as in Test Example 1 and each of them was subjected to an intrabronchial administration.

<Test for gastric excretion >

The rats (body weight used for the experiment: about 200 g) were fasted on the day before the test (before about 24 hours) and were bred only by drinking water. A test for gastric excretion was conducted according to a Phenol Red method [***Arch. Int. Pharmacodyn.***, volume 246, pages 286-294 (1980)].

A 1.5 mL of an 1.5 weight/volume % carboxymethyl cellulose (CMC) solution containing a 0.05 weight/volume % Phenol Red (hereinafter, referred to as P-C solution) was orally administered to the rats where one group comprised six rats. After 15 minutes from the oral administration, the rats were killed and quickly subjected to a laparotomy, and all stomach part including a part of duodenum was extirpated. The extirpated stomach was incised in 40 mL of a 0.1 mol/L aqueous NaOH solution to recover the P-C solution and the amount of Phenol Red remaining in the stomach was measured. Thus, 1 mL of the recovered P-C solution in the stomach was mixed with 2 mL of a 7.5 weight/volume % aqueous trichloroacetic acid (TCA) solution and centrifuged, then 2 mL of the supernatant was mixed with 2 mL of an 1 mol/L aqueous NaOH solution, and absorbance (OD value) of the mixed solution was measured by an autoshipper photometer (560 nm; U-1080; Hitachi, Tokyo). A suspension for administration of Compound (II) or RP 73401 was intrabronchially administered 30 minutes before the oral administration of the P-C solution (agent-administered group). Similarly, a group where the solvent for administration was intrabronchially administered in place of the suspension for administration was prepared (positive control group). Further, a group where the solvent for administration was intrabronchially administered and the content in the stomach was recovered immediately after oral administration of the P-C solution was prepared (negative control group). The gastric excretion ratio (%) in the agent-administered group or the solvent-administered group was calculated by the following equation (1) while the inhibition rate (%) of the agent to the gastric excretion was calculated from the gastric excretion ratio using the following equation (2). The results are shown in Fig. 1.

$$(1) \quad \text{Gastric excretion ratio (\%)} = 1 - \frac{A}{B} \times 100$$

A: OD value of positive control group or agent-administered group
B: Mean value of OD values of negative control group

$$(2) \quad \text{Inhibition ratio (\%) to gastric excretion} = 1 - \frac{C}{D} \times 100$$

C: Gastric excretion ratio in the agent-administered group
D: Mean value of gastric excretion ratio in the negative control group

As a result, when Compound (II) was intrabronchially administered, no significant inhibitory effect to gastric excretion was observed in the effective dose range as shown in Test Example 2 (30 to 100 μg/rat).

On the other hand, similarly, when RP 73401 was intrabronchially administered, a dose-depending inhibitory effect to gastric excretion was observed in the effective dose range as shown in Test Example 2 (30 to 100 μg/rat).

From the above, it is regard that, when Compound (II) is administrated into the airway, vomiting which is a side effect in the conventional PDE-IV inhibitors is able to be avoided.

Thus, as a result of Test Examples 1 to 3, it is shown that pharmaceutical effect in the lung is able to be achieved without causing the influence to the gastrointestinal system by intrabronchial administration of Compound (II) where the ratio of AUC in lung tissues (concentration in lung tissues) to AUC in plasma (concentration in plasma) is 4,970-fold in the case of intrabronchial administration. On the other hand, the effect to the gastrointestinal tracts was caused in the pharmaceutical dose in the respiratory organs by intrabronchial administration of RP 73401 where the ratio of concentration in lung tissues to the concentration in plasma is 300-fold in the case of intrabronchial administration. From those facts, it was confirmed that pharmacological effect in respiratory system diverges from the pharmacological effect in gastrointestinal system being correlated to the ratio of concentration in lung tissues to concentration in plasma.

The agent for intra-airway administration of the present invention comprises a compound having a PDE-IV inhibitory activity or a pharmaceutically acceptable salt thereof where, when it is administered into airway, the concentration in lung tissues is 350-fold or more, preferably 500-fold or more, more preferably 1,000-fold or more and, still more preferably, 2,000-fold or more higher than that in plasma. Although it may be used solely as it is, it is usually provided as various pharmaceutical preparations. Such pharmaceutical preparations are used for animals and human being.

The pharmaceutical preparation according to the present invention is also able to be used as a mixture with one or more of any active ingredient (s) which is/are effective in the other treatment. Such a pharmaceutical preparation is able to be prepared by mixing the active ingredient(s) with one or more pharmaceutically acceptable carrier (s) followed

by subjecting any method which is well known in the art of pharmaceutical sciences.

With regard to an administering route, using the most effective route for the treatment is desired, for example, a method of intra-airway administration such as an inhalation, an intranasal dropping and the like is listed.

With regard to the dosage form, for example, an aerosol preparation, an inhalation preparation, a dry powder preparation and the like are listed.

The aerosol preparation or the inhalation preparation is prepared by making the active ingredient into powder, liquid or suspension, mixing with a propellant for inhalation or a carrier, and charging, for example, in an appropriate inhaler such as metered-dose inhaler and the like. When the above active ingredient is powder, a common mechanical powder inhalator may be used, while when it is liquid or suspension, an inhalator such as a nebulizer and the like may be used. With regard to a propellant for inhalation, conventionally known ones may be widely used and, for example, fron-type compounds such as fron-11, fron-12, fron-21, fron-22, fron-113, fron-114, fron-123, fron-142c, fron-134a, fron-227, fron-C318, 1,1,1,2-tetrafluoroethane and the like, hydrocarbon gases such as propane, isobutene, n-butane and the like, ethers such as diethyl ether, nitrogen gas, carbon dioxide gas and the like are listed. In the case of a preparation in form of suspension, an auxiliary suspending agent such as sorbitan triaurate and the like may be added. With regard to the carrier, conventionally known ones may be widely used and, for example, saccharides, sugar alcohols, amino acids and the like, preferably lactose, D-mannitol and the like are listed.

Dry powder preparation is a preparation for inhalation in the form of powder using a dry powder inhalator, and its characteristic is that the active ingredient being made fine is inhaled as fine particles so that it is able to arrive the airway. It has been known that particle size of the fine active ingredient is preferably within a range of 1 μm to 6 μm [***Int. J. Pharm.,*** volume 101, pages 1-13 (1994)], and with regard to the method for making the active ingredient into fine, a common grinding method may be applied. The grinding method is not particular limited, but the methods which are known in the art are able to be used. For example, mortar grinding, ball mill grinding, hammer mill grinding, liquid energy grinding (such as jet mill grinding) and the like may be listed. The dry powder preparation may also contain carrier particles for suppressing the aggregation of the active ingredient which was made fine and enhancing the arriving rate to the airway. Since the active ingredient which was made fine is adhered on the surface of the carrier particle, the aggregation of the active ingredient is suppressed, and upon inhalation, the active ingredient is separated from the carrier and sent into the airway. With regard to carrier particles, for example, saccharides, sugar alcohols and the like are listed, and lactose, D-mannitol and the like are listed as specific examples. The particle size of the carrier particles is preferably within a range of 20 μm to 150 μm, and more preferably, within a range of 50 μm to 100 μm. The carrier particle may also be subjected to a surface treatment using a ball mill or the like for promoting the separation of active ingredient particles from carrier particles upon inhalation. Amount of the active ingredient in the total weight of the dry powder preparation is preferably 0.5 weight % to 50 weight %, and more preferably, 1.0 weight % to 30 weight %.

Each of the above-mentioned preparations may contain one or more auxiliary ingredient(s) selected from excipients such as lactose, mannitol and the like; disintegrats such as starch and the like; lubricants such as magnesium stearate and the like; binders such as hydroxypropyl cellulose and the like; surfactants such as soybean lecithin, fatty acid ester and the like; plasticizers such as glycerol and the like, and the like.

The dosage and the dosage frequency of the compound having a PDE-IV inhibitory activity or a pharmaceutically acceptable salt thereof used in the present invention may vary depending upon dosage form, age and body weight of a patient, nature or degree of severeness of the symptom to be treated, and the like. Usually, a dose of 1 μg to 1,000 mg, preferably 0.01 to 100 mg, and more preferably, 0.05 to 20 mg per day for an adult is administered once to several times a day. However, the dosage and the dosage frequency vary depending upon the above-mentioned various conditions.

## Brief Description of the Drawing

Fig. 1 shows a relationship between dose of Compound (II) and RP 73401 and the inhibition rate to gastric excretion. An ordinate shows the inhibition rate (%) to the gastric excretion, and an abscissa shows the dose (μg/rat). -o- represents the inhibition rate (%) of Compound (II) to the gastric excretion while -•- represents the inhibition rate (%) of RP 73401 to the gastric excretion.

## Best Mode for Carrying Out the Invention

Although the embodiments of the present invention will be described by way of the following Examples, the scope of the present invention is not limited by those Examples.

### Example 1: Dry powder preparation

Compound (II) (10 g) was ground using a jet mill (A-O Jet; Seishin Kigyo) under air pressure of 5 $kg/cm^2$ and

flowing speed of 1.5 g/minute (volume-average particle size: 5.7 μm). The resulting ground product of Compound (II) and lactose (Pharmatose 325M; registered trade mark, DMV INTERNATIONAL, Veghel, The Netheland) were mixed in a weight ratio of 1: 5 to prepare a dry powder preparation. The resulting preparation is able to be administered by a commonly used dry powder inhalator.

Example 2: Dry powder preparation

The ground product of Compound (II) prepared in Example 1 and lactose (Pharmatose 325M; registered trade mark, DMV INTERNATIONAL 1, Veghel, The Netheland) were mixed in a weight ratio of 1:50 to prepare a dry powder preparation. The resulting preparation is able to be administered by a commonly used dry powder inhalator.

Example 3: Inhalation preparation

The ground product of Compound (II) prepared in Example 1 (100 mg) is suspended in a mixed liquid of 3 mL of liquefied dichlorofluoromethane (fron-21) and 2 mL of liquefied trichlorofluoromethane (fron-11), and charged in a commonly used aerosol nebulizer (nebulizing amount for one shot: 50 μL) to prepare an inhalation preparation.

Example 4: Inhalation preparation

The ground product of Compound (II) prepared in Example 1 (100 mg) and 50 mg of soybean lecithin are suspended in a mixed liquid of 3 mL of liquefied dichlorofluoromethane (fron-21) and 2 mL of liquefied trichlorofluoromethane (fron-11), and charged in a commonly used aerosol nebulizer (nebulizing amount for one shot: 50 μL) to prepare an inhalation preparation.

Example 5: Inhalation preparation

The ground product of Compound (II) prepared in Example 1 (100 mg) is suspended in 100 mg of sorbitan triaurate and 10 g of fron-11. The resulting suspension is dispersed in 50 g of a safe mixed liquid for nebulizing (fron-11/fron-114) at -50°C, and charged in a commonly used aerosol nebulizer to prepare an inhalation preparation.

Example 6: Aqueous suspended inhalation preparation

Compound (II) (1 mg) is dissolved in 5 mL of a mixed solution of water and ethanol (1:1) and filtered through an aseptic Millipore filter (pore size: 0.2 μm) to prepare a preparation for nebulizing. The preparation is able to be administered using a commonly used nebulizer.

Industrial Applicability

An agent for intra-airway administration comprising a compound having a PDE-IV inhibitory activity or a pharmaceutically acceptable salt thereof as an active ingredient, and being able to separate pharmacological effect to the airway system from that to the digestive system.

## Claims

**1.** An agent for intra-airway administration comprising a compound having a PDE-IV inhibitory activity or a pharmaceutically acceptable salt thereof as an active ingredient which shows its concentration in lung tissues 350-times or more higher than its concentration in plasma when administered into the airway.

**2.** An agent for intra-airway administration comprising a compound having a PDE-IV inhibitory activity or a pharmaceutically acceptable salt thereof as an active ingredient which shows its concentration in lung tissues 500-times or more higher than its concentration in plasma when administered into the airway.

**3.** An agent for intra-airway administration comprising a compound having a PDE-IV inhibitory activity or a pharmaceutically acceptable salt thereof as an active ingredient which shows its concentration in lung tissues 1,000-times or more higher than its concentration in plasma when administered into the airway.

**4.** An agent for intra-airway administration comprising a compound having a PDE-IV inhibitory activity or a pharmaceutically acceptable salt thereof as an active ingredient which shows its concentration in lung tissues 2,000-times or more higher than its concentration in plasma when administered into the airway.

5. The agent for intra-airway administration according to any one of claims 1 to 4, wherein the compound having a PDE-IV inhibitory activity is a compound represented by formula (I)

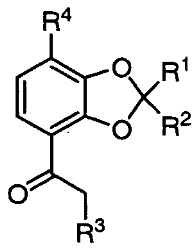

(I)

(wherein, $R^1$ and $R^2$ are the same or different and each represents lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

6. The agent for intra-airway administration according to claim 5, wherein $R^3$ is substituted or unsubstituted pyridyl.

7. The agent for intra-airway administration according to claim 1, wherein the compound having a PDE-IV inhibitory activity is 7-[2-(3,5-dichloro-4-pyridyl)-l-oxoethyl]-4-methoxy-spiro[1,3-benzodioxole-2,1'-cyclopentane] represented by formula (II):

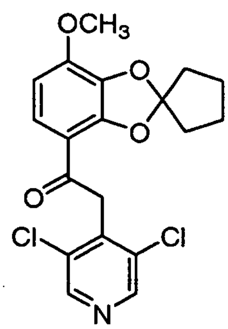

(II)

8. A method of treating and/or preventing a respiratory disease, which comprises administrating an effective amount of a compound having a PDE-IV inhibitory activity or a pharmaceutically acceptable salt thereof into the airway, which shows its concentration in lung tissues 350-times or more higher than its concentration in plasma when administered into the airway.

9. A method of treating and/or preventing a respiratory disease, which comprises administrating an effective amount of a compound having a PDE-IV inhibitory activity or a pharmaceutically acceptable salt thereof into the airway, which shows its concentration in lung tissues 500-times or more higher than its concentration in plasma when administered into the airway.

10. A method of treating and/or preventing a respiratory disease, which comprises administrating an effective amount of a compound having a PDE-IV inhibitory activity or a pharmaceutically acceptable salt thereof into the airway, which shows its concentration in lung tissues 1,000-times or more higher than its concentration in plasma when administered into the airway.

**11.** A method of treating and/or preventing a respiratory disease, which comprises administrating an effective amount of a compound having a PDE-IV inhibitory activity or a pharmaceutically acceptable salt thereof into the airway, which shows its concentration in lung tissues 2,000-times or more higher than its concentration in plasma when administered into the airway.

**12.** The method of treating and/or preventing a respiratory disease according to any one of claims 8 to 11, wherein the compound having a PDE-IV inhibitory activity is a compound represented by formula (I)

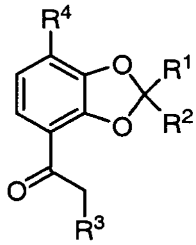

(I)

(wherein, $R^1$ and $R^2$ are the same or different and each represents lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

**13.** The method of treating and/or preventing a respiratory disease according to claim 12, wherein $R^3$ is substituted or unsubstituted pyridyl.

**14.** The method of treating and/or preventing a respiratory disease according to claim 8, wherein the compound having a PDE-IV inhibitory activity is 7-[2-(3,5-dichloro-4-pyridyl)-1-oxoethyl]-4-methoxy-spiro-[1,3-benzodioxole-2,1'-cyclopentane] represented by formula (II):

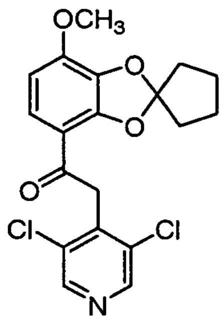

(II)

**15.** The method of treating and/or preventing a respiratory disease according to any one of claims 8 to 14, wherein the respiratory disease is a disease selected from the group consisting of bronchial asthma, chronic obstructive pulmonary disease (COPD), pulmonary emphysema, chronic bronchitis, pulmonary fibrosis, pulmonary hypertension and eosinophilic pneumonia.

**16.** Use of a compound having a PDE-IV inhibitory activity or a pharmaceutically acceptable salt thereof which shows its concentration in lung tissues 350-times or more higher than its concentration in plasma when administered into the airway, for the manufacture of an agent for intra-airway administration.

**17.** Use of a compound having a PDE-IV inhibitory activity or a pharmaceutically acceptable salt thereof which shows its concentration in lung tissues 500-times or more higher than its concentration in plasma when administered into the airway, for the manufacture of an agent for intra-airway administration.

**18.** Use of a compound having a PDE-IV inhibitory activity or a pharmaceutically acceptable salt thereof which shows its concentration in lung tissues 1,000-times or more higher than its concentration in plasma when administered into the airway, for the manufacture of an agent for intra-airway administration.

**19.** Use of a compound having a PDE-IV inhibitory activity or a pharmaceutically acceptable salt thereof which shows its concentration in lung tissues 2,000-times or more higher than its concentration in plasma when administered into the airway, for the manufacture of an agent for intra-airway administration.

**20.** Use according to any one of claims 16 to 19, wherein the compound having a PDE-IV inhibitory activity is a compound represented by formula (I)

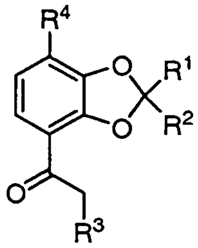

(I)

(wherein, $R^1$ and $R^2$ are the same or different and each represent lower alkyl, or $R^1$ and $R^2$, together with the adjacent carbon atom, form a saturated carbon ring; $R^3$ represents a substituted or unsubstituted aromatic heterocyclic group; and $R^4$ represents hydroxy or lower alkoxy).

**21.** Use according to claim 20, wherein $R^3$ is substituted or unsubstituted pyridyl.

**22.** Use according to claim 16, wherein the compound having a PDE-IV inhibitory activity is 7-[2-(3,5-dichloro-4-pyridyl)-1-oxoethyl]-4-methoxy-spiro-[1,3-benzodioxole-2,1'-cyclopentane] represented by formula (II):

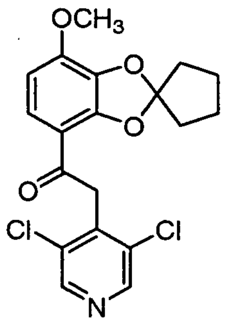

(II)

Fig. 1

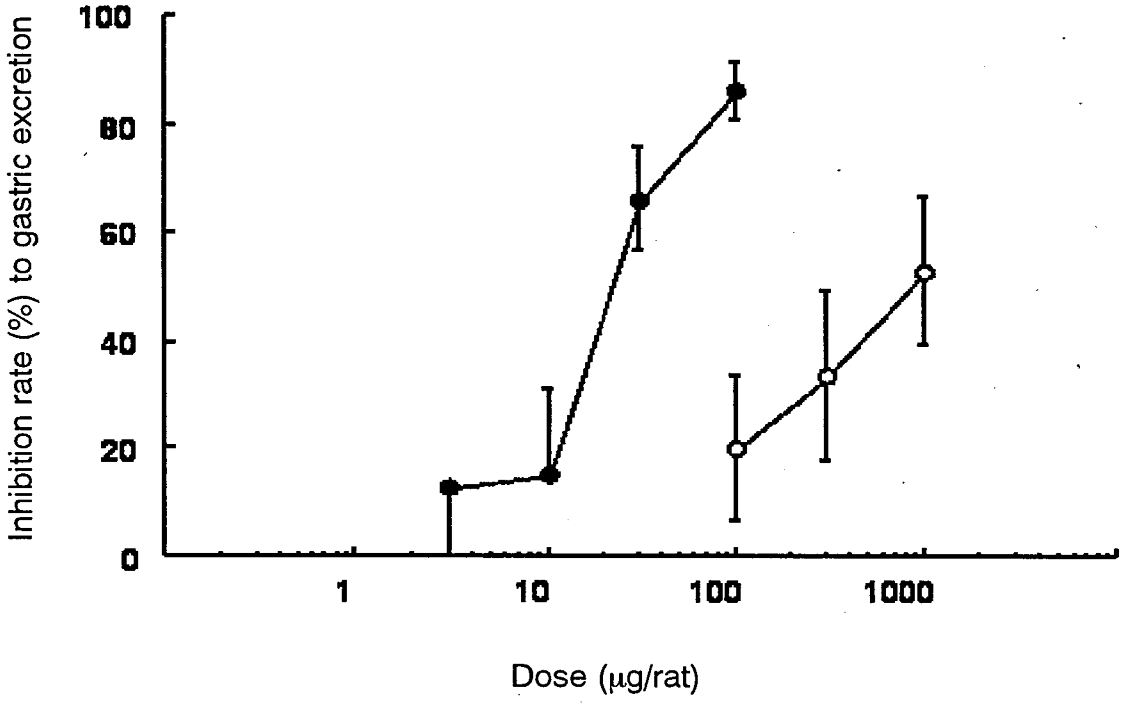
Inhibition rate (%) to gastric excretion
100
80
60
40
20
0
1
10
100
1000
Dose (μg/rat)

**INTERNATIONAL SEARCH REPORT**

International application No. PCT/JP2004/004601

**A. CLASSIFICATION OF SUBJECT MATTER**

Int.Cl[7] A61K45/00, A61K31/443, A61P11/00//C07D405/06

According to International Patent Classification (IPC) or to both national classification and IPC

**B. FIELDS SEARCHED**

Minimum documentation searched (classification system followed by classification symbols)

Int.Cl[7] A61K45/00-45/08, A61K31/00-31/80, A61P1/00-43/00, C07D405/00-405/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2004 |
| Kokai Jitsuyo Shinan Koho | 1971-2004 | Toroku Jitsuyo Shinan Koho | 1994-2004 |

Electronic data base consulted during the international search (name of data base and, where practicable, search terms used)

MEDLINE(STN), EMBASE(STN), BIOSIS(STN), BIOTECHABS(STN), CAplus(STN), REGISTRY(STN), WPI(DIALOG), JSTPLUS(JOIS), JMEDPLUS(JOIS)

**C. DOCUMENTS CONSIDERED TO BE RELEVANT**

| Category* | Citation of document, with indication, where appropriate, of the relevant passages | Relevant to claim No. |
|---|---|---|
| X | WO 96/36624 A1 (Kyowa Hakko Kogyo Co., Ltd.), 21 November, 1996 (21.11.96), Claims; example 140; page 61, line 15 to page 63, line 3 | 1-7,16-22 |
| P,X | WO 03/066044 A1 (BOERINGER INGELHEIM PHARM GMBH & CO. KG), 14 August, 2003 (14.08.03), Example 140; description, page 26, line 26 to page 27, line 2 | 1-7,16-22 |
| P,A | WO 2004/005276 A1 (Kyowa Hakko Kogyo Co., Ltd.), 15 January, 2004 (15.01.04) | 1-7,16-22 |
| A | WO 01/32165 A1 (SMITHKLINE BEECHAM CORP.), 10 May, 2001 (10.05.01) | 1-7,16-22 |

[X] Further documents are listed in the continuation of Box C. [X] See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| | |
|---|---|
| Date of the actual completion of the international search<br>16 June, 2004 (16.06.04) | Date of mailing of the international search report<br>13 July, 2004 (13.07.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/004601

C (Continuation). DOCUMENTS CONSIDERED TO BE RELEVANT

| Category* | Citation of document, with indication, where appropriate, of the relevant passages | Relevant to claim No. |
|---|---|---|
| A | WO 03/016279 A1 (Tanabe Seiyaku Co., Ltd.), 27 February, 2003 (27.02.03) | 1-7,16-22 |
| A | WO 02/098880 A1 (BAYER AG.), 12 December, 2002 (12.12.02) | 1-7,16-22 |
| A | JP 2002-537383 A (Merck Frosst Canada and Co.), 05 November, 2002 (05.11.02) | 1-7,16-22 |

Form PCT/ISA/210 (continuation of second sheet) (January 2004)

**INTERNATIONAL SEARCH REPORT**

International application No.
PCT/JP2004/004601

**Box No. II Observations where certain claims were found unsearchable (Continuation of item 2 of first sheet)**

This international search report has not been established in respect of certain claims under Article 17(2)(a) for the following reasons:

1. [X] Claims Nos.: 8-15
because they relate to subject matter not required to be searched by this Authority, namely:
The inventions according to claims 8 to 15 pertain to methods for treatment of the human body by therapy (PCT Article 17(2)(a)(i) and PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**
Information on patent family members

International application No.
PCT/JP2004/004601

| | | |
|---|---|---|
| WO 96/36624 A1 | 1996.11.21 | AU 9657029 A<br>NO 9700151 A<br>EP 771794 A1<br>KR 97704724 A<br>US 2002/0128290 A1<br>US 6514996 B2<br>CN 1154697 A<br>US 671697 B1 |
| WO 03/016279 A1 | 2003.02.27 | JP 2003-119180 A<br>JP 2003-119195 A<br>JP 2003-119196 A<br>EP 1424326 A1 |
| WO 01/32165 A1 | 2001.05.10 | AU 200113445 A<br>NO 200201937 A<br>BR 200015039 A<br>EP 1225884 A1<br>KR 2002050249 A<br>SK 200200729 A3<br>CZ 200201443 A3<br>CN 200201443 A3<br>HU 200203682 A2<br>ZA 200203349 A<br>US 2003/0212113 A1<br>MX 2002004220 A1<br>NZ 518002 A |
| WO 02/098880 A1 | 2002.12.12 | EP 1397363 A1 |
| JP 2002-538115 A | 2002.11.12 | WO 00/51599 A1<br>AU 200037150 A<br>EP 1156800 A1<br>NO 200104221 A<br>BR 200008625 A<br>KR 2001102460 A<br>HU 200200026 A2<br>CN 1350453 A<br>CZ 200103148 A3<br>ZA 20010187 A<br>UA 6555576 B1<br>MX 200100085 A1 |

Form PCT/ISA/210 (patent family annex) (January 2004)

INTERNATIONAL SEARCH REPORT

International application No. PCT/JP2004/004601

Claims 1 to 7 and 16 to 22

The inventions according to claims 1 to 7 and 16 to 22 are characterized in that, in the case of administering a compound having a PDE-IV inhibitory effect or its salt into the airway, the ratio of its concentration in a lung tissue to that in the plasma remains at a definite level or higher. Even though the description is examined, however, it is not specifically illustrated how to achieve such a ratio. Considering EXAMPLES, it is merely stated that a drug containing a compound having the chemical structure represented by the formula (II) in claim 7 satisfies the above ratio. It cannot be considered that this fact is self-evident for a person skilled in the art.

Concerning the inventions according to claims 1 to 7 and 16 to 22, therefore, the statement in the description does not comply with the prescribed requirements to such an extent extent that no meaningful international search can be carried out.

As discussed above, the statement of the description does not comply with the prescribed requirements. In this international search report, therefore, prior art was searched exclusively relating to the case where the compound administered in practice into the airway in EXAMPLE was employed as a compound having a PDE-IV effect.